# NEUE EUROPÄISCHE PATENTSCHRIFT

(11) **EP 1 023 049 B2**
(45) Veröffentlichungstag und Bekanntmachung des Hinweises auf die Entscheidung über den Einspruch: **20.10.2010**
(45) Hinweis auf die Patenterteilung: 29.11.2006
(21) Anmeldenummer: 98949002.4
(22) Anmeldetag: 02.10.1998
(51) Int. Cl.: A61K 9/20

(54) **HERSTELLUNG EINES DIREKT VERPRESSBAREN TABLETTIERHILFSSTOFFES**
PREPARATION OF A DIRECTLY MOULDABLE TABLETTING AUXILIARY
PREPARATION D'UN AUXILIAIRE D'AGGLOMERATION DIRECTEMENT COMPRESSIBLE

(30) Priorität: 15.10.1997 DE 19745265
(43) Veröffentlichungstag der Anmeldung: 02.08.2000
(73) Patentinhaber: Merck Patent GmbH, 64271 Darmstadt (DE)
(72) Erfinder: SCHWARZ, Eugen, D-64625 Bensheim (DE); MÖSCHL, Gernot, D-64331 Weiterstadt (DE); MAUL, Karin, White Plains, New York 10605 (US)
(86) Internationale Anmeldenummer: PCT/EP1998/006272
(87) Internationale Veröffentlichungsnummer: WO 1999/018935

(56) Entgegenhaltungen:
- EP-A- 0 306 454
- EP-A- 0 329 977
- EP-A- 0 490 768
- EP-A1- 0 275 834
- WO-A-90/14821
- WO-A-92/10168
- WO-A-98/22094
- WO-A1-91//07100
- WO-A1-96//14282
- DE-A- 4 439 858
- FR-A- 2 336 123
- US-A- 5 139 795
- US-A- 5 158 789
- US-A- 5 536 526
- S.Z. DZIEDZIC, M.W. KEARSLEY: 'Glucose Syrups: Science and Technology', teil CHPT.3 1984 Seiten 117 - 135
- G.G. BIRCH, K.J. PARKER: 'Nutritive Sweeteners', teil CHPT.8 1982 Seiten 145 - 170

## Beschreibung

Die Erfindung betrifft direkt verpreßbare Tablettierhilfsstoffe mit einem Xylit-Gehalt größer als 90 Gew.-% und einem Gehalt an mindestens einem weiteren Polyol kleiner als 10 Gew.-%, die durch Co-Sprühtrocknung hergestellt werden. Die erfindungsgemäßen Tablettierhilfsstoffe besitzen im Vergleich zu Xylit verbesserte Tablettiereigenschaften, insbesondere hinsichtlich der resultierenden Tablettenhärten, der Abriebsneigung und der Tendenz zum Deckeln. Diese verbesserten Tablettiereigenschaften der erfindungsgemäßen Tablettierhilfsstoffe treten insbesondere bei Formulierungen mit einem hohen Anteil an Wirkstoffen auf. Zudem weisen die erfindungsgemäßen Tablettierhilfsstoffe im Vergleich zu bekannten Polyolen verbesserte geschmacksmaskierende Eigenschaften auf und beeinflussen das sensorische Mundgefühl der hergestellten Produkte in vorteilhafter Weise. Die Erfindung betrifft weiterhin Zusammensetzungen, Formulierungen und feste Formen bzw. Komprimate, die einen erfindungsgemäßen Tablettierhilfsstoff enthalten sowie ein Verfahren zur Herstellung der erfindungsgemäßen Tablettierhilfsstoffe.

Polyole und Polyolmischungen werden in großem Umfang als Zusatzstoffe und Trägerstoffe unter anderem für pharmazeutische Wirkstoffe, Kau- und Lutschtabletten und andere Produkte der Pharmaindustrie und als Komprimate in der Lebensmittelindustrie verwendet. Aufgrund seiner vorteilhaften Eigenschaften besteht ein besonderes Interesse daran, Xylit als Tablettierhilfsstoff zu verwenden. Unter anderem besitzt Xylit süßende Eigenschaften, die vergleichbar mit denen von Saccharose sind. Vorteilhafterweise ist es jedoch nicht kariogen. Es gibt sogar Hinweise darauf, daß Xylit imstande sein soll, Karies vorzubeugen. Zusätzlich zeigt Xylit beim Auflösungsprozeß einen als angenehm empfundenen Kühleffekt.

Bei der Herstellung von Komprimaten durch direkte Verpressung ergeben viele Polyole eine wenig zufriedenstellende Oberfläche oder sie führen zu Komprimaten mit einer unbefriedigenden Härte. So zeigen die bekannten Polyole Mannit, Lactit, Isomalt und Xylit ein schlechtes Tablettierverhalten, was in einer geringen Tablettenhärte, Deckeln und einem hohen Abrieb der Tabletten resultiert. Insbesondere Xylit führt beim direkten Verpressen zu äußerst unbefriedigenden Ergebnissen.

Sollen derartige Polyole trotzdem zur Komprimatherstellung verwendet werden, muß dies in der Regel mit dem Nachteil eines erhöhten Arbeitsaufwandes erkauft werden. Dies wird am Beispiel von Mannit deutlich. Mannit wird trotz der obengenannten Nachteile im Gegensatz zu Lactit, Isomalt und Xylit, die in der Komprimatherstellung eher ungebräuchlich sind, in pharmazeutischen Formulierungen durchaus verwendet. Mannit muß jedoch in der Regel vor dem Verpressen mit den übrigen Bestandteilen granuliert oder brikettiert werden.

Der Einsatz von Polyolgemischen zur Herstellung von Xylit-haltigen Komprimaten ist bekannt. Allerdings ist der Xylit-Anteil in der Regel relativ gering. In der EP 0 528 604 A1 wird beispielsweise eine durch Co-Schmelzen erhältliche Zusammensetzung aus Sorbit und Xylit beschrieben, die besonders bevorzugt ein Gewichtsverhältnis Sorbit: Xylit im Bereich von 65 : 35 bis 95 : 5 enthält. Da in diesen Zusammensetzungen ein großer Teil des Xylits durch Sorbit ersetzt ist, wird nur ein Bruchteil der vorteilhaften Eigenschaften von Xylit genutzt.

In der EP 0 329 977 B1 werden Binde- und Verdünnungsmittel beansprucht, die 94 bis 98 Gew.-% Xylit enthalten und für die Darstellung von direkt verpreßten Tabletten geeignet sind. Allerdings wird zur Herstellung dieser Binde- und Verdünnungsmittel von kristallinem Xylit ausgegangen, wodurch u.a. eine erhöhte Anzahl von Arbeitsschritten erforderlich wird.

Es besteht daher ein Interesse an vereinfachten Verfahren zur Herstellung von direkt verpreßbaren Polyolgemischen auf Basis von Xylit.

In der DE 44 39 858 A1 wird vorgeschlagen, eine Polyolkombination durch Sprühtrocknung herzustellen, die im wesentlichen aus mindestens zwei Polyolen mit einem Mannit-Gehalt von weniger als 10 Gew.-% besteht. Dadurch sollen problemlos herstellbare Polyol-Zusammensetzungen, deren Tablettiereigenschaften und Plastizität gegenüber bekannten Polyolen oder Polyolkombinationen verbessert ist, zur Verfügung gestellt werden. Als bevorzugte Zusammensetzungen werden solche Zusammensetzungen beschrieben, die Sorbit und Xylit oder Sorbit, Xylit und weitere Polyole, insbesondere Sorbit, Xylit und Mannit als Polyole enthalten. Besonders bevorzugt ist der Xylit-Gehalt kleiner als 50 Gew.-%, insbesondere bevorzugt kleiner als 35 Gew.-%. Es wurde unter anderem gefunden, daß die hergestellten Polyol-Zusammensetzungen beim Tablettieren viel glattere Oberflächen ergeben und daß sich diese Produkte zu Kaugummis verarbeiten lassen, welche bessere Verarbeitungseigenschaften aufweisen als die mit herkömmlichem Sorbit oder Mischungen aus Sorbit und weiteren Polyolen hergestellten Kaugummi. Es findet sich in der DE 44 39 858 A1 jedoch kein Hinweis darauf, daß mit durch Sprühtrocknung erhältlichen Polyolkombinationen mit einem höheren Xylit-Gehalt, insbesondere mit einem Xylit-Gehalt, der größer als 90 Gew.-% ist, direkt verpreßbare Tablettierhilfsstoffe auf Basis des üblicherweise sehr schlecht direkt verpreßbaren Xylits erhalten werden können, die zudem weitere günstige Eigenschaften, insbesondere eine Geschmacksmaskierung bei einer Co-Sprühtrocknung oder Co-Wirbelschichtgranulation mit Wirkstoffen und eine vorteilhafte Beeinflussung des sensorischen Mundgefühls der hergestellten Produkte, aufweisen.

Bei der Formulierung oral zu verabreichender pharmazeutischer Zusammensetzungen bereitet in vielen Fällen nicht nur bei flüssigen Applikationsformen das vom Verwender empfundene Geschmacksbild Probleme. Insbesondere beim Kauen von Antacidum-Tabletten wird ein kreidiger, seifiger Geschmack als unangenehm empfunden. Es wurde bislang wenig erfolgreich durch verschiedenste Zusatzstoffe versucht, diesen unangenehmen Geschmack zu überdecken.

Als problematisch hat sich auch bei verschiedensten Wirkstoffen ein als äußerst bitter empfundener Geschmack erwiesen. Eine Maskierung der besonders bitter schmeckenden Wirkstoffe ist bisher auch durch den Zusatz von Geschmacks- oder Aromastoffen nicht gelungen. Es besteht zwar die Möglichkeit, entsprechende Wirkstoffe enthaltende Tabletten mit einem Überzug zu versehen, diese Methode ist jedoch ungeeignet, wenn eine schnelle Aufnahme des Wirkstoffs, die bereits beim Kauen der Tabletten über die Mundschleimhaut erfolgt, angestrebt wird.

Besondere Anforderungen werden auch an die Oberfläche von Tabletten gestellt, die gelutscht werden sollen, wie beispielsweise Halstabletten. Erwünscht ist hierbei eine glatte Oberfläche der eigentlichen Tablette, die während des Lutschens erhalten bleibt und sich nicht nach und nach aufrauht.

Weiterhin werden heute im Bereich der Nahrungsergänzung (Vitamin- und Mineralstoffsupplementierung) vermehrt Lutsch- und vor allem Kautabletten angeboten. Insbesondere bei Tabletten zur Mineraistoffanreicherung ist der Trägeranteil sehr gering, so daß das Geschmacksbild weitgehend vom entsprechenden Mineralstoff geprägt wird.

Es bestand daher die Aufgabe, einen direkt verpreßbaren Tablettierhilfsstoff zur Verfügung zu stellen, der einfach herstellbar ist und der im Vergleich zu Xylit verbesserte Tablettiereigenschaften, insbesondere hinsichtlich der resultierenden Tablettenhärten, der Abriebsneigung sowie der Tendenz zum Deckeln besitzt, und im Vergleich zu bekannten Polyolen zusätzlich verbesserte geschmacksmaskierende Eigenschaften aufweist und das sensorische Mundgefühl der hergestellten Produkte vorteilhaft beeinflußt.

Es wurde nun gefunden, daß die oben genannte Aufgabe der vorliegenden Erfindung gelöst werden kann, wenn der Tablettierhilfsstoff Anspruch 1 entspricht.

Gegenstand der Erfindung ist somit ein einfach herstellbarer direkt verpreßbarer Tablettierhilfsstoff, gemäß Anspruch 1 der die folgenden Eigenschaften aufweist:
- im Vergleich zu Xylit verbesserte Tablettiereigenschaften, insbesondere hinsichtlich der resultierenden Tablettenhärten, der Abriebsneigung sowie der Tendenz zum Deckeln
- im Vergleich zu bekannten Polyolen verbesserte geschmacksmaskierende Eigenschaften und
- vorteilhafte Beeinflussung des sensorischen Mundgefühls der hergestellten Produkte.

Der Begriff "Polyol" steht für Zuckeralkohole der allgemeinen Formel

HOCH₂-(CHOH)ₙ-CH₂OH,

wobei n für 2 bis 6, vorzugsweise 3 bis 4, steht,
sowie deren dimere Anhydride, insbesondere C₁₂H₂₄O₁₁.

Insbesondere steht der Begriff "Polyole" für Hexite wie Sorbit (zum Vergleich) und Mannit, Pentite wie Xylit, möglich sind aber auch C₄-Polyalkohole wie Erythrit oder C₁₂-Polyalkohole wie Lactit, Maltit oder lsomalt. Neben Polyolen können aber auch geeignete andere Kohlehydrate eingesetzt werden.

Bevorzugte Ausführungsformen sind:
a1) direkt verpreßbare Tablettierhilfsstoffe, erhältlich durch Lösen von Xylit und mindestens einem weiteren Polyol nach Anspruch 1 in Wasser und Versprühen des erhaltenen wäßrigen Gemisches in einem Luftstrom bei einer Temperatur von 120 °C bis 300 °C.
c) direkt verpreßbare Tablettierhilfsstoffe, wobei das Verhältnis von Xylit zu Mannit in einem Bereich zwischen 90 : 10 bis 98 : 2, insbesondere zwischen 90 : 10 bis 95 : 5 liegt.
d) direkt verpreßbare Tablettierhilfsstoffe, wobei das Verhältnis von Xylit zu Lactit in einem Bereich zwischen 90 : 10 bis 98 : 2, insbesondere zwischen 90 : 10 bis 95 : 5 liegt.
e) direkt verpreßbare Tablettierhilfsstoffe, wobei das Verhältnis Xylit : Mannit: Lactit in einem Bereich zwischen 90 : 1 : 9 bzw. 90 : 9 : 1 und 98 : 1 : 1 liegt.
f) direkt verpreßbare Tablettierhilfsstoffe nach einer der vorangehenden bevorzugten Ausführungsformen a1) bis e), wobei der Gehalt an Wasser niedriger als 1 Gew.-% ist.

Ein weiterer Gegenstand der Erfindung sind Zusammensetzungen, Formulierungen und feste Formen bzw. Komprimate, enthaltend einen erfindungsgemäßen Tablettierhilfsstoff.

Die zur Herstellung der festen Formen bzw. Komprimate eingesetzte Gesamtmenge Polyol ist so zu wählen, daß 10 Gew.-% bis 99 Gew.-%, insbesondere 25 Gew.-% bis 98 Gew.-% Polyol in den erfindungsgemäßen festen Formen bzw. Komprimaten enthalten ist.

In diesen festen Formen bzw. Komprimaten können einerseits Mineralstoffe aus der Gruppe physiologisch verträglicher Ca-, Mg-, Na-, K-, Fe- und Zn-Salze in einer Menge von 10 Gew.-% bis 90 Gew.-%, insbesondere von 25 Gew.-% bis 75 Gew.-%, gegebenenfalls Spurenelemente, sowie ein oder mehrere Vitamine und gegebenenfalls ein oder mehrere eventuell bitter schmeckende Wirkstoffe enthalten sein.

In den festen Formen bzw. Komprimaten können ein oder mehrere pharmazeutische Wirkstoffe enthalten sein. Solche Wirkstoffe können unter anderem Analgetica, Antacida oder andere sein. Die pharmazeutischen Wirkstoffe können in einer Menge von 0,1 Gew.-% bis 75 Gew.-% enthalten sein.

Die erfindungsgemäßen Tablettierhilfsstoffe eignen sich auch zur Herstellung von geformten und ungeformten Polyolmassen, die durch Schmelzextrusion hergestellt werden. Diese können wiederum Wirkstoffe bis zu einem Gehalt von 80 Gew.-% enthalten.

Natürlich sind die im vorhergehenden Text angegebenen Gew.-%-Angaben so zu verstehen, daß die Gewichtsprozente der eingesetzten Substanzen in der Summe 100 Gew.-% nicht übersteigen.

Weiterhin Gegenstand der Erfindung ist ein Verfahren zur Herstellung der erfindungsgemäßen Tablettierhilfsstoffe, beinhaltend die folgenden Schritte:
a) Herstellen einer wäßrigen Lösung aus Xylit und mindestens einem weiteren Polyol, wobei das erhaltene Gemisch einen Xylit-Gehalt größer als 90 Gew.-% bezogen auf den Gesamtpolyolgehalt enthält,
b1) Versprühen des erhaltenen Gemisches in einem Luftstrom bei einer Temperatur von 120°C bis 300 °C, wobei das Wasser verdampft wird,
b2) Verwirbeln des erhaltenen Gemisches in einem Luftstrom bei einer Temperatur von 30 °C bis 110 °C, wobei das Wasser verdampft wird, und
c) Isolierung des Tablettierhilfsstoffes.

In einer besonders bevorzugten Ausführungsform besteht der erfindungsgemäße Tablettierhilfsstoff aus 90 bis 98 Gew.-%, insbesondere 90 bis 95 Gew.-% Xylit, und 2 bis 10 Gew.-%, insbesondere 5 bis 10 Gew.-% aus einem oder zwei Polyolen ausgewählt aus Mannit und Lactit.

Ganz besonders bevorzugt enthält der erfindungsgemäße Tablettierhilfsstoff mehr als 95 Gew.-% Xylit und weniger als 5 Gew.-% eines Polyols ausgewählt aus Mannit und Lactit.

Zur Co-Sprühtrocknung wird eine wäßrige Lösung aus Xylit und mindestens einem weiteren Polyol verwendet. Der Feststoffgehalt wird zuvor vorzugsweise durch Mischen bei einer Temperatur von bis zu 80 °C zweier oder mehrerer Polyol-Lösungen im gewünschten Verhältnis auf etwa 30 Gew.-% bis etwa 75 Gew.-%, insbesondere 60 Gew.-% bis 72 Gew.-% eingestellt. Die Versprühung wird durch Zerstäuben mittels Düsen, vorzugsweise mittels eines Zentrifugalzerstäubers in einen auf eine Temperatur von 120 °C bis 300 °C, vorzugsweise 130 °C bis 190 °C erwärmten trockenen zentrifugal eingeblasenen Luftstrom durchgeführt. Die Menge der zugeführten Polyollösung und der eingeblasenen Heißluft wird so abgestimmt, daß das Substanzgemisch bis auf einen Wassergehalt von etwa 0,1 Gew.-% bis etwa 1 Gew.-%, gegebenenfalls im Fließbett, getrocknet wird. Auf jeden Fall sollte der Wassergehalt unterhalb 1 Gew.-% liegen.

Die Polyolteilchen, die dabei durch Entwässerung der Polyollösungströpfchen erhalten werden, werden bei der Sprühtrocknung auf eine Temperatur von etwa 50 °C bis etwa 70 °C erwärmt, während sich die eingeblasene Luft auf etwa die gleiche Temperatur abkühlt. Die Polyolzusammensetzung wird in Behältem gesammelt und ist nach dem Abkühlen direkt zur Herstellung von Tabletten oder Komprimaten geeignet.

Die Co-Wirbelschichtgranulation wird, wie beispielsweise in P. Grassmann, F. Widmer, "Einführung in die thermische Verfahrenstechnik", Verlag DeGruyter, Berlin 1974, beschrieben, durchgeführt.

Dem wäßrigen Gemisch können vor der Co-Sprühtrocknung beispielsweise ein oder mehrere Wirkstoffe hinzugefügt werden. Pharmazeutische Wirkstoffe können unter anderem Analgetica, Antacida oder andere sein. Desweiteren können dem wäßrigen Gemisch vor der Sprühtrocknung beispielsweise auch geschmackskorrigierende Substanzen und gegebenenfalls Farbstoffe hinzugefügt sein. Als geschmackskorrigierende Substanzen kommen u.a. natürliche oder synthetische Süßstoffe aus der Gruppe Saccharin, Aspartam^{®}, Acesulfam K, Neohesperidin DC, Sucralose, Thaumatin oder Steviosid in Frage.

Aufgrund der besonderen Herstellungsart durch Versprühen einer wäßrigen Lösung ist es möglich, nicht wasserlösliche und wasserlösliche Zusätze, wie z.B. Zitronensäure, Süßstoffe, insbesondere Acesulfam K, Aspartam^{®}, Saccharin, Cyclamat, Sucralose, Neohesperidin DC, Farbstoffe sowie pharmazeutische Wirkstoffe, wie z.B. Analgetika, Antacida und dergleichen, Vitamine, Mineralstoffe und gegebenenfalls Spurenelemente homogen in den erfindungsgemäßen Zusammensetzungen bzw. Formulierungen und den daraus hergestellten festen Formen bzw. Komprimaten, insbesondere den daraus hergestellten Tabletten zu verteilen. Derartige feste Formen bzw. Komprimate sind ebenfalls Gegenstand der Erfindung.

Die gegebenenfalls zuzusetzenden Bindemittel sind dem Fachmann geläufig und dienen der Erhöhung der Festigkeit der Zusammensetzung. Als Bindemittel bevorzugt sind Cellulose-Derivate, insbesondere Hydroxypropylmethylcellulose, Carboxymethylcellulose oder Stärke.

Neben der erfindungsgemäßen Polyol-Zusammensetzung enthalten die erfindungsgemäßen Komprimate einen oder mehrere Bestandteile augewählt aus pharmazeutischen Wirkstoffen und lebensmittelrechtlich zugelassenen Stoffen. Bevorzugte lebensmittelrechtlich zugelassene Stoffe sind natürliche, naturidentische oder künstliche Aroma- oder Geschmacksstoffe, Vitamine, Spurenelemente, Mineralien, Farbstoffe, Gleitmittel, Trennmittel, Süßstoffe, Stabilisatoren oder Antioxidantien. Der Anteil dieser Bestandteile liegt vorzugsweise zwischen 0,01 und 90 Gew.-% und insbesondere zwischen 0,1 und 70 Gew.-%.

Die Herstellung der Komprimate erfolgt in an sich bekannter Weise durch Vermischen der Bestandteile in trockener Form und anschließender Tablettierung.

Die erfindungsgemäßen Polyol-Zusammensetzungen besitzen eine Reihe von vorteilhaften Tablettiereigenschaften:
Überraschenderweise kann festgestellt werden, daß durch das erfindungsgemäße Verfahren unter Verwendung der erfindungsgemäßen Zusammensetzungen feste Formen, insbesondere Tabletten, mit einem erheblich verbesserten Geschmacksbild und sensorischen Mundgefühl erhalten werden. Bei Verwendung von Formulierungen mit einem hohen Mineralstoffgehalt von bis zu 90 Gew.-% werden einerseits drastisch verbesserte Tablettiereigenschaften gefunden, andererseits sind die hergestellten Tabletten während des Verpackungsvorgangs durch einen wesentlich geringeren Abrieb gekennzeichnet. Auch werden bei Verwendung der erfindungsgemäßen Zusammensetzungen bei gleicher Preßkraft, wie sie bei bekannten polyolhaltigen Formulierungen angelegt wird, härtere Tabletten mit glatteren Oberflächen erhalten. Dieses anfänglich empfundene verbesserte sensorische Gefühl im Mund wird auch beim Kauen oder Lutschen empfunden, da der sonst übliche kreidige, oder gegebenenfalls seifige Geschmack weitestgehend überdeckt ist. Überraschenderweise wird jedoch nicht nur das Geschmacksbild dieser Mineralstofftabletten verbessert. Auch Formulierungen, in die äußerst bitter schmeckende Wirkstoffe eingearbeitet sind, werden als wesentlich wohlschmeckender empfunden, da der Bittergeschmack nicht mehr so extrem durchschlägt.

Die nachfolgenden Beispiele dienen der besseren Veranschaulichung der beschriebenen und beanspruchten Erfindung. Sie sind jedoch keinesfalls als Einengung des Schutzbereichs auf diese Beispiele zu verstehen.

### Beispiele

Es wurde ein Vergleich des Tablettierverhaltens von
(1) co-sprühgranuliertem Xylit in Verbindung mit weiteren Polyolen (Beispiele 1 bis 4),
(2) Xylit-Qualitäten des Handels (Vergleichsbeispiel 1) und
(3) sprühgranuliertem Rein-Xylit (Vergleichsbeispiel 2)
durchgeführt.

### Tablettierung:

wenn nicht explizit anders beschrieben, werden jeweils ca. 1000 Tabletten aus insgesamt ca. 500 g Material hergestellt;
Gerät: Exzenter-Tablettenpresse EKO DMS (instrumentiert); Fa. Korsch

### Messungen:

- Tabletten-Härte:
   es werden 20 Tabletten vermessen und der Mittelwert gebildet; Gerät: Bruchfestigkeitstester 6D, Fa. Schleuniger
- Abrieb:
   es werden 20 Tabletten vermessen und eine Rückwägung vorgenommen;
      Gerät: Friabilator, Fa. RWK
- Tabletten-Gewicht:
   es werden 20 Tabletten vermessen und der Mittelwert gebildet;
      Gerät: Mettler AT 201 mit Statistikprogramm und Drucker LCP 45, Fa. Mettler

### Beispiele 1 bis 4

Xylit (Hersteller: Fa. Cerestar) wurde mit Zusätzen von 5 - 10 Gew.-% eines weiteren Polyols aufgelöst und einer Sprühgranulation unterzogen. Die Sprühgranulation wurde wie oben beschrieben durchgeführt. Anschließend wurde die Tablettierfähigkeit der sprühgranulierten Materialien getestet. Es wurden 1000 Tabletten aus einem Granulat hergestellt.

Zum Vergleich wurden mechanische Mischungen der Ausgangskomponenten auf ihre Tablettierfähigkeit untersucht. Auch hier wurden 1 000 Tabletten aus einer mechanischen Mischung hergestellt.

Es wurden Tabletten mit einem Durchmesser von 11 mm hergestellt und ein Tabletten-Gewicht von 500 mg angestrebt.

Wenn nicht explizit anders beschrieben, wurden jeweils 20 Tabletten vermessen und geprüft.

### Beispiel 1

Vergleich der Tablettierfähigkeit von Co-sprühgetrocknetem Xylit (Zusatz: 5 Gew-% Lactit (Hersteller: Fa. Purac)) und der Tablettierfähigkeit einer mechanischen Mischung gleicher Zusammensetzung

**Tabelle 1 Meßwerte zu Beispiel 1**

| | Co | Me | Co | Me | Co | Me | Co | Me |
|---|---|---|---|---|---|---|---|---|
| Preßdruck [kN] | 4,5 | 4,5 | 10 | * | 21 | 21 | 32 | 30 |
| Tabletten-Härte [N] | 20 | 10 | 43 | * | 98 | 34 | 131 | 30 |
| Abrieb [Gew.-%] | 0,44 | Ze | 0,11 | * | 0,08 | 65 | 0,06 | 90 |
| Tabletten-Gewicht [mg] | 502 | 498 | 503 | * | 502 | 503 | 502 | 501 |

| | | | | | | | | |
|---|---|---|---|---|---|---|---|---|
| Co: Co-Sprühtrocknung Me: mechanische Mischung *: starkes Rauhlaufen - Tablettierung nicht möglich Ze: Zerfall der Tablette | | | | | | | | |

### Beispiel 2

Vergleich der Tablettierfähigkeit von Co-sprühgetrocknetem Xylit (Zusatz: 5 Gew-% Mannit (Hersteller: Fa. Merck KGaA)) und der Tablettierfähigkeit einer mechanischen Mischung gleicher Zusammensetzung

**Tabelle 2 Meßwerte zu Beispiel 2**

| | Co | Me | Co | Me | Co | Me | Co | Me |
|---|---|---|---|---|---|---|---|---|
| Preßdruck [kN] | 5 | 5 | 9,5 | 11 | 20 | 21,5 | 32 | 30 |
| Tabletten-Härte [N] | 51 | <20 | 76 | <20 | 95 | <20 | 95 | <20 |
| Abrieb [Gew.-%] | 2,0 | 37 | 0,72 | 3,8 | * | Ze | * | Ze |

| | | | | | | | | |
|---|---|---|---|---|---|---|---|---|
| Co: Co-Sprühtrocknung Me: mechanische Mischung *: nicht bestimmt (häufiges Deckeln) Ze: Zerfall der Tablette | | | | | | | | |

Das Tabletten-Gewicht wurde nicht bestimmt.

### Beispiel 3

Vergleich der Tablettierfähigkeit von Co-sprühgetrocknetem Xylit (Zusatz: 10 Gew-% Mannit (Hersteller: Fa. Merck KGaA)) und der Tablettierfähigkeit einer mechanischen Mischung gleicher Zusammensetzung

**Tabelle 3 Meßwerte zu Beispiel 3**

| | Co | Me | Co | Me | Co | Me | Co | Me |
|---|---|---|---|---|---|---|---|---|
| Preßdruck [kN] | 4,5 | 5 | 10 | 10 | 19,5 | 19 | 29 | 31 |
| Tabletten-Härte [N] | 30 | <20 | 63 | <20 | 96 | <20 | 108 | <20 |
| Abrieb [Gew.-%] | 3,2 | 11 | 0,53 | 6,5 | 0,44 | Ze | 0,67 | Ze |
| Tabletten-Gewicht [mg] | 501 | 500 | 502 | 500 | 502 | 501 | 501 | 501 |

| | | | | | | | | |
|---|---|---|---|---|---|---|---|---|
| Co: Co-Sprühtrocknung Me: mechanische Mischung Ze: Zerfall der Tablette | | | | | | | | |

### Beispiel 4 zum Vergleich

Vergleich der Tablettierfähigkeit von Co-sprühgetrocknetem Xylit (Zusatz: 5 Gew-% Sorbit (Hersteller: Fa. Merck KGaA)) und der Tablettierfähigkeit einer mechanischen Mischung gleicher Zusammensetzung

**Tabelle 4 Meßwerte zu Beispiel 4**

| | Co | Me | Co | Me |
|---|---|---|---|---|
| Preßdruck [kN] | 21 | 20 | 31 | 30 |
| Tabletten-Härte [N] | 85 | 34 | 83 | 37 |
| Abrieb [Gew.-%] | 0,18 | 31 | 0,12 | 21 |
| Tabletten-Gewicht [mg] | 501 | 501 | 498 | 498 |

| | | | | |
|---|---|---|---|---|
| Co: Co-Sprühtrocknung Me: mechanische Mischung | | | | |

Bei niedrigeren Preßdücken war aufgrund eines starken Rauhlaufens keine Tablettierung möglich.

Die Beispiele 1 bis 3 zeigen, daß die Tabletten, die aus sprühgranuliertem Xylit hergestellt werden, deutlich bessere Eigenschaften besitzen als die Tabletten, die aus der Verpressung der entsprechenden mechanischen Mischungen hervorgehen. Die Co-Sprühtrocknung führt insbesondere zu wesentlich höheren Tabletten-Härten und deutlich geringerem Abrieb. Nach der Erfindung hergestellter cosprühgranulierter Xylit ist für eine direkte Tablettierung sehr gut geeignet.

### Vergleichsbeispiel 1

Es wurden 5 marktgängige Xylit-Qualitäten hinsichtlich ihrer Tablettierfähigkeit getestet (Hersteller: Fa. Cerestar, Fa. Roquette, Fa. Finnsugar: jeweils eine Probe; Fa. Xyrofin: zwei Proben)

Die angegebenen Meßwerte des Vergleichsbeispiels 1 sind die Mittelwerte für die Meßwerte über alle 5 Proben.

Für die Beispiele wurde ein einheitlicher Preßdruck von 20 kN bei einem Tablettendurchmesser von 11 mm angestrebt. Bei geringerem Preßdruck konnte das zu verpressende Material kaum verfestigt werden. Bei höherem Preßdruck trat Deckeln und ein Rückgang der Festigkeit der Preßlinge auf.

**Tabelle V1 Meßwerte zu Vergleichsbeispiel 1**

| | Mittelwert | Bereich der Meßwerte | Sᵣₑₗ. |
|---|---|---|---|
| Tablettendurchmesser [mm] | 11 | - | - |
| Preßdruck [kN] | 20 | 18 - 21 | n.b. |
| Tabletten-Härte [N] | 31,5 | 27 - 39 | 5 |
| Abrieb [Gew.-%] | 12 | 4 - 24 | 8 |

| | | | |
|---|---|---|---|
| S_{rel.}: relative Standardabweichung n.b.: nicht bestimmt | | | |

Das Tabletten-Gewicht wurde zahlenmäßig nicht erfaßt, da sehr starke, nicht tolerierbare Schwankungen innerhalb der einzelnen Xylit-Musterqualitäten auftraten. Zudem erschien die Angabe der Meßdaten nicht sinnvoll, da die einzelnen Muster verschiedene Komstrukturen aufweisen.

Bei der Tablettierung kam es oftmals zum Rauhlaufen und zum Deckeln in der Tablettiermaschine.

Die Tablettierversuche zeigen, daß alle 5 Xylit-Qualitäten für eine direkte Tablettierung ungeeignet sind.

### Vergleichsbeispiel 2

Herkömmlicher Xylit (Hersteller: Fa. Cerestar) wurde ohne weitere Zusätze aufgelöst und einer Sprühgranulation unterzogen. Die Sprühgranulation wurde wie oben beschrieben durchgeführt. Es wurden 1000 Tabletten aus einem Granulat hergestellt.

**Tabelle V2 Meßwerte zu Vergleichsbeispiel 2**

| | Sprühgranulierung im Zerstäubungstrockner | Sprühgranulierung in der Wirbelschicht |
|---|---|---|
| Tablettendurchmesser [mm] | 11 | 11 |
| Preßdruck [kN] | 20 | 20 |
| Tabletten-Härte [N] | 57 (Bereich: 52 - 62) | 60 (Bereich: 49 - 71) |
| Abrieb [Gew.-%] | 10,5 | 3 |
| Tabletten-Gewicht [mg] | 510 | 460 |

Die Meßwerte zeigen, daß sprühgranulierter Rein-Xylit ohne weitere Zusätze nicht tablettierbar ist. Für eine Tablette von 11 mm Durchmesser sind die Tabletten-Härten zu gering und der Abrieb ist zu hoch. Das Tabletten-Gewicht ist zudem innerhalb einer Meßreihe starken Schwankungen unterlegen.

## Patentansprüche

1. Direkt verpreßbarer Tablettierhilfsstoff mit einem Xylit-Gehalt größer als 90 Gew.-% und einen Gehalt an mindestens einem weiteren Polyol kleiner als 10 Gew.-%, erhältlich durch Lösen von Xylit und mindestens einem weiteren Polyol in Wasser und anschließende Sprühtrocknung enthaltend Xytit und Mannit, Xytit und Lactit oder Xylit, Mannit und Lactit als Polyole.

2. Direkt verpreßbarer Tablettierhilfsstoff gemäß Anspruch 1, enthaltend Xylit und mindestens ein Polyol ausgewählt aus der Gruppe Mannit und Lactit.

3. Direkt verpreßbarer Tablettierhilfsstoff nach einem der Ansprüche 1 oder 2, erhältlich durch Lösen von Xylit und mindestens einem weiteren Polyol in Wasser und Versprühen des erhaltenen wässrigen Gemisches in einem Luftstrom bei einer Temperatur im Bereich von 120 °C bis 300 °C.

4. Direkt verpreßbarer Tablettierhilfsstoff nach Anspruch 1, **dadurch gekennzeichnet, dass** das Verhältnis von Xylit zu Mannit in einem Bereich zwischen 90 : 10 bis 98 : 2, insbesondere zwischen 90 : 10 bis 95 : 5 liegt.

5. Direkt verpreßbarer Tablettierhilfsstoff nach Anspruch 1, **dadurch gekennzeichnet, dass** das Verhältnis von Xylit zu Lactit in einem Bereich zwischen 90: 10 bis 98 : 2, insbesondere zwischen 90:10 bis 95: 5 liegt.

6. Direkt verpreßbarer Tablettierhilfsstoff nach Anspruch 1, **dadurch gekennzeichnet, dass** das Verhältnis Xylit: Mannit: Lactit in einem Bereich zwischen 90 : 1 : 9 bzw. 90 : 9 : 1 und 98 : 1 : 1 liegt.

7. Direkt verpreßbarer Tablettierhilfsstoff nach Anspruch 1, **dadurch gekennzeichnet, dass** der Gehalt an Wasser niedriger als 1 Gew.-% ist.

8. Verfahren zur Herstellung eines direkt verpreßbaren Tablettierhilfsstoffes nach einem der Ansprüche 1 - 7, **dadurch gekennzeichnet, dass** es die folgenden Schritte beinhaltet:
a) Herstellen einer wässrigen Lösung aus Xylit und mindestens einem Polyol, wobei das erhaltene Gemisch einen Xylit-Gehalt größer als 90 Gew.-% bezogen auf den Gesamtpolyolgehalt enthält,
b1) Versprühen des erhaltenen Gemischs in einem Luftstrom bei einer Temperatur von 120 °C bis 300 °C, wobei das Wasser verdampft,
b2) Verwirbeln des erhaltenen Gemischs in einem Luftstrom bei einer Temperatur von 30 °C bis 110 °C, wobei das Wasser verdampft wird
und
c) Isolierung des Tablettierhilfsstoffs.

9. Verwendung eines direkt verpreßbaren Tablettierhilfsstoffs nach einem der Ansprüche 1 bis 7 zur Herstellung von geformten und ungeformten Polyolmassen durch Schmelzextrusion.

10. Zusammensetzungen bzw. Formulierungen, **dadurch gekennzeichnet, dass** sie einen direkt verpreßbaren Tablettierhilfsstoff nach einem der Ansprüche 1 bis 7 enthalten.

11. Feste Formen bzw. Komprimate, **dadurch gekennzeichnet, dass** sie einen direkt verpreßbaren Tablettierhilfsstoff nach einem der Ansprüche 1 bis 7 enthalten.

12. Feste Formen bzw. Komprimate nach Anspruch 11, **dadurch gekennzeichnet, dass** sie einen oder mehrere nicht wasserlösliche und/oder wasserlösliche Zusätze homogen verteilt enthalten.

13. Feste Formen bzw. Komprimate nach einem der Ansprüche 11 oder 12, **dadurch gekennzeichnet, dass** sie als Zusatz Zitronensäure enthalten.

14. Feste Formen bzw. Komprimate nach einem der Ansprüche 11 bis 13, **dadurch gekennzeichnet, dass** sie einen oder mehrere Zusätze ausgewählt aus der Gruppe pharmazeutische Wirkstoffe, Süßstoffe, Farbstoffe, Vitamine und Spurenelemente enthalten.

15. Feste Formen bzw. Komprimate nach Anspruch 14, **dadurch gekennzeichnet, dass** sie einen oder mehrere pharmazeutische Wirkstoffe aus-gewählt aus der Gruppe Analgetica und Antacida enthalten.

16. Direkt verpreßbarer Tablettierhilfsstoff nach Anspruch 1, **dadurch gekennzeichnet, dass** sie einen oder mehrere Süßstoffe ausgewählt aus der Gruppe Acesulfam K, Aspartam®, Saccharin, Cyclamat, Sucralose und Neohesperidin DC enthalten.

## Claims

1. Directly compressible tabletting auxiliary having a xylitol content of greater than 90% by weight and a content of at least one further polyol of less than 10% by weight, obtainable by dissolution of xylitol and at least one further polyol in water and subsequent spray-drying, comprising xylitol and mannitol, xylitol and lactitol, or xylitol, mannitol and lactitol as polyols.

2. Directly compressible tabletting auxiliary according to Claim 1, comprising xylitol and at least one polyol selected from the group mannitol and lactitol.

3. Directly compressible tabletting auxiliary according to one of Claims 1 or 2, obtainable by dissolution of xylitol and at least one further polyol in water and spraying the resultant aqueous mixture in a stream of air at a temperature in the range from 120°C to 300°C.

4. Directly compressible tabletting auxiliary according to Claim 1, **characterised in that** the ratio of xylitol to mannitol is in a range between 90 : 10 and 98 : 2, in particular between 90 : 10 and 95 : 5.

5. Directly compressible tabletting auxiliary according to Claim 1, **characterised in that** the ratio of xylitol to lactitol is in a range between 90 : 10 and 98 : 2, in particular between 90 : 10 and 95 : 5.

6. Directly compressible tabletting auxiliary according to Claim 1, **characterised in that** the xylitol : mannitol : lactitol ratio is in a range between 90 : 1 : 9 or 90 : 9 : 1 and 98 : 1 : 1.

7. Directly compressible tabletting auxiliary according to Claim 1, **characterised in that** the water content is less than 1% by weight.

8. Process for the preparation of a directly compressible tabletting auxiliary according to one of Claims 1-7, **characterised in that** it includes the following steps:
a) preparation of an aqueous solution of xylitol and at least one polyol, where the resultant mixture has a xylitol content of greater than 90% by weight, based on the total polyol content,
b1) spraying of the resultant mixture in a stream of air at a temperature of 120°C to 300°C, during which the water evaporates,
b2) fluidising the resultant mixture in a stream of air at a temperature of 30°C to 110°C, during which the water is evaporated, and
c) isolation of the tabletting auxiliary.

9. Use of a directly compressible tabletting auxiliary according to one of Claims 1 to 7 for the preparation of shaped and unshaped polyol compositions by melt extrusion.

10. Compositions or formulations, **characterised in that** they comprise a directly compressible tabletting auxiliary according to one of Claims 1 to 7.

11. Solid shapes or compressed products, **characterised in that** they comprise a directly compressible tabletting auxiliary according to one of Claims 1 to 7.

12. Solid shapes or compressed products according to Claim 11, **characterised in that** they comprise one or more homogeneously distributed water-insoluble and/or water-soluble additives.

13. Solid shapes or compressed products according to one of Claims 11 or 12, **characterised in that** they comprise citric acid as additive.

14. Solid shapes or compressed products according to one of Claims 11 to 13, **characterised in that** they comprise one or more additives selected from the group pharmaceutical active ingredients, sweeteners, dyes, vitamins and trace elements.

15. Solid shapes or compressed products according to Claim 14, **characterised in that** they comprise one or more pharmaceutical active ingredients selected from the group analgesics and antacids.

16. Directly compressible tabletting auxiliary according to Claim 1, **characterised in that** it comprises one or more sweeteners selected from the group acesulfame K, Aspartame^{®}, saccharine, cyclamate, sucralose and neohesperidin DC.

## Revendications

1. Auxiliaire de formation de comprimés directement compressible présentant une teneur en xylitol supérieure à 90% en poids et une teneur en au moins un autre polyol inférieure à 10% en poids, pouvant être obtenu par dissolution de xylitol et d'au moins un autre polyol dans de l'eau et par un séchage par pulvérisation qui suit, comprenant xylitol et mannitol, xylitol et lactitol, ou xylitol, mannitol et lactitol en tant que polyols.

2. Auxiliaire de formation de comprimés directement compressible selon la revendication 1, comprenant du xylitol et au moins un polyol qui est choisi parmi le groupe comprenant mannitol et lactitol.

3. Auxiliaire de formation de comprimés directement compressible selon l'une des revendications 1 ou 2, pouvant être obtenu par dissolution de xylitol et d'au moins un autre polyol dans de l'eau et par pulvérisation du mélange aqueux résultant dans une circulation d'air à une température dans la plage de 120°C à 300°C.

4. Auxiliaire de formation de comprimés directement compressible selon la revendication 1, **caractérisé en ce que** le rapport de xylitol sur mannitol est dans une plage entre 90 : 10 et 98 : 2, en particulier entre 90 : 10 et 95 : 5.

5. Auxiliaire de formation de comprimés directement compressible selon la revendication 1, **caractérisé en ce que** le rapport de xylitol sur lactitol est dans une plage entre 90 : 10 et 98 : 2, en particulier entre 90 : 10 et 95 : 5.

6. Auxiliaire de formation de comprimés directement compressible selon la revendication 1, **caractérisé en ce que** le rapport de xylitol : mannitol : lactitol est dans une plage entre 90 : 1 : 9 ou 90 : 9 : 1 et 98 : 1 : 1.

7. Auxiliaire de formation de comprimés directement compressible selon la revendication 1, **caractérisé en ce que** la teneur en eau est inférieure à 1% en poids.

8. Procédé pour la préparation d'un auxiliaire de formation de comprimés directement compressible selon l'une des revendications 1-7, **caractérisé en ce qu'**il inclut les étapes qui suivent :
a) préparation d'une solution aqueuse de xylitol et d'au moins un polyol, où le mélange résultant présente une teneur en xylitol supérieure à 90% en poids, sur la base de la teneur totale en polyol,
b1) pulvérisation du mélange résultant dans une circulation d'air à une température de 120°C à 300°C, pulvérisation pendant laquelle l'eau s'évapore,
b2) fluidisation du mélange résultant dans une circulation d'air à une température de 30°C à 110°C, fluidisation pendant laquelle l'eau est évaporée, et
c) isolation de l'auxiliaire de formation de comprimés.

9. Utilisation d'un auxiliaire de formation de comprimés directement compressible selon l'une des revendications 1 à 7 pour la préparation de compositions de polyol conformées et non conformées par extrusion de matière fondue.

10. Compositions ou formulations, **caractérisées en ce qu'**elles comprennent un auxiliaire de formation de comprimés directement compressible selon l'une des revendications 1 à 7.

11. Formes solides ou produits comprimés, **caractérisés en ce qu'**ils comprennent un auxiliaire de formation de comprimés directement compressible selon l'une des revendications 1 à 7.

12. Formes solides ou produits comprimés selon la revendication 11, **caractérisés en ce qu'**ils comprennent un ou plusieurs additifs non solubles à l'eau et/ou solubles à l'eau distribués de façon homogène.

13. Formes solides ou produits comprimés selon l'une des revendications 11 ou 12, **caractérisés en ce qu'**ils comprennent de l'acide citrique en tant qu'additif.

14. Formes solides ou produits comprimés selon l'une des revendications 11 à 13, **caractérisés en ce qu'**ils comprennent un ou plusieurs additifs choisis parmi le groupe d'ingrédients actifs pharmaceutiques, d'édulcorants, de colorants, de vitamines et d'oligoéléments.

15. Formes solides ou produits comprimés selon la revendication 14, **caractérisés en ce qu'**ils comprennent un ou plusieurs ingrédients actifs pharmaceutiques choisis parmi le groupe des analgésiques et des antiacides.

16. Auxiliaire de formation de comprimés directement compressible selon la revendication 1, **caractérisé en ce qu'**il comprend un ou plusieurs édulcorants choisis parmi le groupe comprenant acésulfame K, Aspartame^{®}, saccharine, cyclamate, sucralose et néohespéridine DC.
